# EUROPÄISCHE PATENTSCHRIFT

(11) **EP 1 084 963 B1**
(45) Veröffentlichungstag und Bekanntmachung des Hinweises auf die Patenterteilung: **29.12.2004**
(21) Anmeldenummer: 00890273.6
(22) Anmeldetag: 05.09.2000
(51) Int. Cl.: B01L 3/00, G01N 33/00, G01N 27/416, B65D 81/20

(54) **Verfahren und Vorrichtung zur Verbesserung der Lagerfähigkeit tonometrierter Flüssigkeiten**
Method and device for improving the storage stability of tonometric fluids
Procédé et dispositif pour améliorer la stabilité au stockage de fluides tonométriques

(30) Priorität: 13.09.1999 AT 157099
(43) Veröffentlichungstag der Anmeldung: 21.03.2001
(73) Patentinhaber: F.HOFFMANN-LA ROCHE AG, 4070 Basel (CH)
(72) Erfinder: Rüther, Horst, Dipl.-Ing. Dr., 8047 Hart/Graz (AT); List, Helmut, Dipl.-Ing. Prof., 8010 Graz (AT)
(74) Vertreter: Babeluk, Michael, Dipl.-Ing.

(56) Entgegenhaltungen:
- WO-A-90/08308
- DE-A- 2 708 337
- US-A- 4 116 336
- US-A- 5 690 215
- US-A- 5 780 302
- US-A- 5 913 232
- PATENT ABSTRACTS OF JAPAN vol. 015, no. 477 (P-1283), 4. Dezember 1991 (1991-12-04) & JP 03 205533 A (TERUMO CORP), 9. September 1991 (1991-09-09)

## Beschreibung

Die Erfindung betrifft ein Verfahren zur Verbesserung der Lagerfähigkeit tonometrierter Flüssigkeiten mit zumindest einer gelösten Gaskomponente, sowie einen Behälter aus einem flexiblen, gasdichten Material, welcher sich beim Befüllen entfaltet und beim Entleeren kollabiert, vorzugsweise ein Aluminium-Mehrschichtlaminatbeutel zur Aufnahme und Lagerung tonometrierter Flüssigkeiten.

Für die Kalibration von Gassensoren können die entsprechenden Gase verwendet werden oder auch tonometrierte Flüssigkeiten, welche die entsprechenden Gase in gelöster Form enthalten. Sollen neben den Gassensoren in einer gemeinsamen Sensoranordnung auch andere Sensoren (z.B. Elektrolytsensoren, pH-Sensoren, Substratsensoren, etc.) kalibriert werden, und sollen bei der Kalibration gleiche Bedingungen wie bei der Messung von flüssigen Proben realisiert werden, dann empfiehlt sich die Verwendung von tonometrierten Flüssigkeiten.

Diese können beispielsweise vor Ort durch Tonometrie mittels Präzisionsgasen aus Gasflaschen hergestellt werden. Diese bereits über Jahrzehnte angewandte Praxis weist jedoch den Nachteil auf, dass separate Gasflaschen erforderlich sind, welche regelmäßig zu tauschen sind und strengen Sicherheitsbestimmungen unterliegen.

Als Alternative bieten sich vortonometrierte Flüssigkeiten an, welche ohne die oben erwähnten Nachteile verwendet werden können. Allerdings besteht hierbei die Problematik in der Lagerung von begasten Flüssigkeiten, insbesondere dann, wenn die Gaskomponente keine chemische Bindung eingeht sondern nur in gelöster Form vorliegt.

Es ist seit langem bekannt, derartige tonometrierte Flüssigkeiten in Glasampullen oder auch Metalldosen zu lagern, wobei während der Lagerung der Flüssigkeit die bei der Befüllung eingestellten Konzentrationswerte an Inhaltsstoffen ohne größere Abweichungen aufrecht erhalten werden können. Probleme treten allerdings während der Phase der Flüssigkeitsentnahme beim Einsatz in den zu kalibrierenden Geräten auf, da die starren Behälter eine Belüf tungsöffnung benötigen um die Flüssigkeit entnehmen zu können. Durch den Gaszutritt wird die Gasphase über der Flüssigkeit verändert, wodurch sich auch die chemische Zusammensetzung der Flüssigkeit bzw. die darin gelösten Gase ändern.

Die oben geschilderte Situation kann durch die Verwendung von flexiblen Behältern, beispielsweise von Kunststoffbeuteln aus Folienmaterial, verbessert werden. So ist beispielsweise aus der US A 4,116.336 ein Beutel aus flexiblem Material bekannt geworden, welcher eine Referenzflüssigkeit für einen Blutgasanalysator enthält. Unter Vermeidung einer Gasphase enthält der flexible, gasdichte Behälter eine Flüssigkeit, welche bei einer vorgegebenen Temperatur bekannte Werte für pH, PCO₂ und PO₂ enthält. Der Partialdruck der Gase liegt unter 600 mmHG bei 37°C. Die Referenzflüssigkeit gemäß US A 4,116.336 kann nicht lösliche, organische Substanzen, z.B. Fluorocarbone enthalten, um die Sauerstofflöslichkeit und dadurch die Gesamtmenge an Sauerstoff zu erhöhen. Der flexible Beutel besteht im wesentlichen aus einem mehrschichtigen Folienmaterial, welches an den Rändern verschweißt ist.

Aus der WO 97/16 309 ist weiters ein flexibler Beutel mit einer Sauerstoff-Referenzlösung bekannt, wobei hier ebenfalls ein mehrlagiges Folienmaterial für den Beutel Verwendung findet. Die innere, der Referenzlösung zugewandte Schicht der Folie, besteht aus einem verschweißbaren Polymerfilm, die mittlere Lage aus Aluminium und die äußere Lage aus Polyester. Die Lagerfähigkeit der Kalibrierlösung wird für Raumtemperatur (25°C) mit 61 Wochen angegeben, wobei eine Abweichung von 2 % von den Anfangswerten angegeben wird. Die Veränderung der Gaswerte ist jedoch logarithmisch von der Außentemperatur abhängig. So reduziert sich beispielsweise die Lagerfähigkeit bei 50°C auf ca. 1,3 Wochen, wobei zu beachten ist, dass derartige Temperaturen im Wege des Transportes (Lufttransport, Lagerhallen, etc.) durchaus nichts Außergewöhnliches darstellen. Ein ähnliches Produkt, bekannt als "Cal B" von Mallinckrodt Sensor Systems Inc., Ann Arbor, MI, weist bei 25°C nur eine Lagerdauer von 7 Wochen auf.

Weiters ist aus der WO 93/21533 A1 ein flexibler Behälter bekannt, welcher ein Kalibriermittel mit gelösten Gasen beinhaltet. Zur Stabilisierung der Konzentration der gelösten Gase wird Helium verwendet.

Aus der EP 0 724 152 A2 ist ein Behälter zur Aufnahme von Kalibrierflüssigkeiten und/oder anderer Betriebsflüssigkeiten für die Blutgasanalyse bekannt. Der in einer Ausführungsvariante dargestellte Behälter aus einem elastischen, gasdichten Material ist mit einem selbstdichtenden Verbindungselement versehen, welches mit einem Anschlussdorn (Hohlnadel) des Analysators zusammenwirkt.

Weiters zeigt die WO 86/05590 A1 eine Vorrichtung zur Messung von Blutparametern. Es werden zwei tonometrierte Referenzflüssigkeiten verwendet, für welche flexible, gasimpermeabler Beutel ohne Gasraum vorgesehen sind.

Schließlich ist aus der JP 03-205 533 A2 eine Vorrichtung mit zwei flexiblen Beuteln bekannt. Das Gesamtsystem steht aus einem Innenbeutel, welcher mit einer Kalibrierflüssigkeit gefüllt ist und von einem mit einer Gasphase gefüllten, gasdichten Außenbeutel umschlossen ist. Der Innenbeutel weist eine definierte Durchlässigkeit für CO₂ und O₂ auf. Da der Außenbeutel mit einer Gasphase gefüllt ist, kann aus dem Umgebungsdruck (außerhalb des Beutelsystems) nicht auf den Innendruck in der Flüssigkeit geschlossen werden. Damit ist eine Berechnung der Tonometriewerte nicht möglich. Weitere Nachteile ergeben sich bei der Produktion, wobei ein Innen- und ein Außenbeutel hergestellt, separat gefüllt und separat verschlossen werden müssen.

Ein flexibler Beutel zur Aufnahme einer Flüssigkeit mit einer darin gelösten Gaskomponente ist weiters aus der US 5,690,215 A bekannt. Ein flexibler, gasdichter Innenbeutel aus einem Mehrschichtlaminat beinhaltet die Flüssigkeit und wird von einem gasdichten Außenbeutel umschlossen, wobei im Zwischenraum zwischen Innen- und Außenbeutel eine Gasphase vorgesehen ist, welche das in der Flüssigkeit gelöste Gas enthält. Es ergeben sich die bereits anhand der JP 03-205 533 A2 beschriebenen Nachteile.

Flexible, kalibrierbare Beutel haben weiters den Nachteil, nicht absolut gasdicht zu sein. Zusätzlich können auch Folienoxidationen auftreten, welche ebenfalls für eine Verfälschung der O₂-Werte verantwortlich sind. Weiters besteht bei der Lagerung von tonometrierten Reagenzien in den bekannten flexiblen Beuteln die Gefahr, der Bildung von Mikrobläschen in der Flüssigkeit, wenn die Beutel bei einem Druck unterhalb des Abfülldruckes gelagert werden (siehe z.B. US-A 4,116,336).

Aufgabe der vorliegenden Erfindung ist es, ein Verfahren zur Erhöhung der Lagerstabilität von tonometrierten Flüssigkeiten in flexiblen Behältern vorzuschlagen bzw. flexible Behälter für tonometrierte Flüssigkeiten derart weiterzubilden, dass die Problematik der Bildung von Mikrobläschen bewältigt werden kann.

Diese Aufgabe wird erfindungsgemäß dadurch gelöst, dass in den flexiblen Behälter zusätzlich eine zumindest die gelöste Gaskomponente enthaltende Gasphase eingebracht wird, wobei die Gasphase von der tonometrierten Flüssigkeit durch deren Flüssigkeitsspiegel getrennt ist und wobei das Volumen der tonometrierten Flüssigkeit und das Volumen der Gasphase in Summe kleiner ist als das maximale Füllvolumen des flexiblen Behälters.

Während bei bekannten Verfahren mit flexiblen Behältern eine Gasphase vermieden wird, um den einfachen physikalisch-chemischen Zusammenhang zwischen Partialdruck, Löslichkeit und Konzentration zur Bestimmung des Partialdruckes verwenden zu können, wird dieses Konzept bei der Erfindung fallen gelassen um eine zusätzliche Gasphase mit einem deutlich höheren Gasanteil, als dies in der wässrigen Flüssigkeit möglich ist, in den flexiblen Behälter einbringen zu können. Da der flexible Behälter nicht zur Gänze gefüllt ist, übt die Behälterfolie in jedem Füll- bzw. Entleerungsstadium eine vernachlässigbare Kraft auf den Beutelinhalt aus, so dass beispielsweise bei einem Dreifach-Laminat-Beutel nur eine vernachlässigbare Druckdifferenz von kleiner 1 mbar zwischen Innen- und Außendruck festgestellt werden kann. Dies führt dazu, dass mit Hilfe des Außendruckes (Barometerdruck), welcher im wesentlichen dem Innendruck entspricht und der Temperatur der Partialdruck des bzw. der in der Flüssigkeit gelösten Gase bestimmt werden kann.

Ein erfindungsgemäßer Behälter aus einem flexiblen, gasdichten Material, welcher eine tonometrierte Flüssigkeit mit zumindest einer gelösten Gaskomponente enthält, zeichnet sich somit dadurch aus, dass die tonometrierte Flüssigkeit einen ersten Volumsanteil beansprucht, dass der flexible Behälter eine einen zweiten Volumsanteil beanspruchende Gasphase aufweist, welche zumindest die in der Flüssigkeit gelöste Gaskomponente enthält, wobei die Gasphase von der tonometrierten Flüssigkeit durch deren Flüssigkeitsspiegel getrennt ist, sowie dass der erste und der zweite Volumsanteil in Summe kleiner sind als das maximale Füllvolumen des flexiblen Behälters. Das Verhältnis der Volumsanteile der tonometrierten Flüssigkeit und der Gasphase kann dabei im Bereich von 1:3 bis 3:1, vorzugsweise bei ca. 1:1 liegen.

Falls nur gelöste Gase tonometriert gelagert werden sollen, dann sollte das Gasvolumen im Vergleich zum Flüssigkeitsvolumen möglichst groß sein, um eine gute Pufferung zu erhalten. Falls neben gelösten Gasen, z.B. O₂, auch chemisch gebundene Gase, z.B. CO₂, tonometriert gelagert werden, dann müssen in der Regel größere Flüssigkeitsvolumen verwendet werden. Es ist somit, abhängig vom Anwendungsfall, ein Optimum der Volumsverhältnisse zu berechnen.

Bei der Befüllung der Kunststoffbeutel können diesen beispielsweise exakt abgemessene Gasund Flüssigkeitsvolumina zugeführt werden. In einer einfachen Ausgestaltung der Erfindung ist es allerdings auch möglich, dass der flexible Behälter in eine die Entfaltung des Behälter bei einem vorgegebenen Volumen begrenzende Vorrichtung eingesetzt wird, dass eine abgemessene Menge der tonometrierten Flüssigkeit in den Behälter gefüllt wird und dass anschließend die Gasphase eingebracht wird, wobei sich der flexible Behälter bis zum vorbestimmten Volumen entfaltet. Vorteilhafterweise beträgt dabei das vorgegebene Volumen 30-90%, vorzugsweise 60-75% des maximalen Füllvolumens.

Die Erfindung wird im folgenden anhand von Zeichnungen näher erläutert. Es zeigen
- Fig. 1: einen erfindungsgemäßen flexiblen Behälter in einer Seitenansicht,
- Fig. 2: den flexiblen Behälter gemäß Fig. 1 in einer Außenkonturform ebenfalls in einer Seitenansicht,
- Fig. 3: eine Abfüllanlage für den flexiblen Behälter, sowie
- Fig. 4: die graphische Darstellung der Lagerfähigkeit in Abhängigkeit der Lagertemperatur.

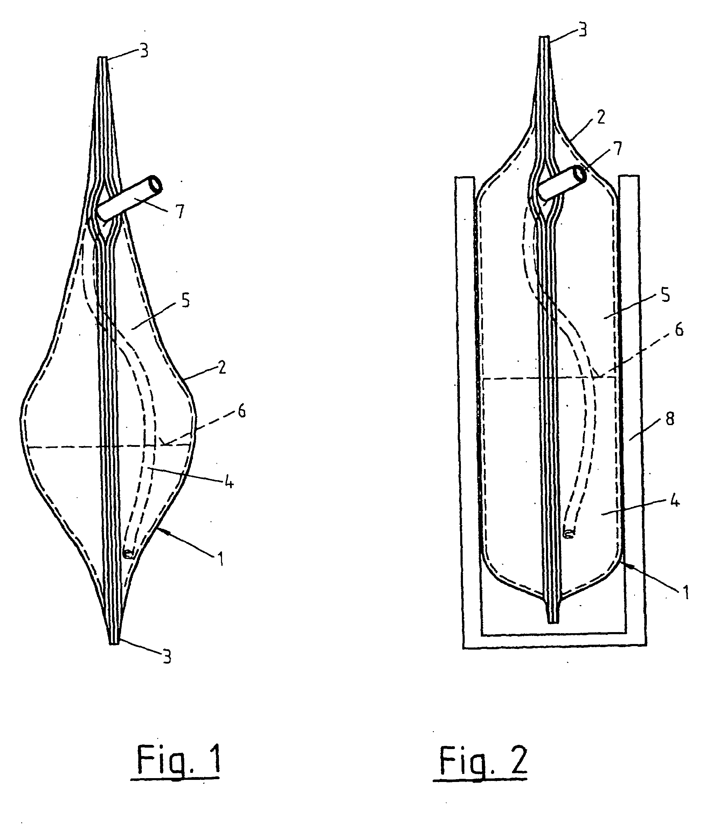

Der in Fig. 1 und Fig. 2 dargestellte flexible Behälter 1 besteht aus einem Folienmaterial 2, welches an den Rändern 3 verschweißt ist. Das Folienmaterial ist mehrlagig und sollte ein möglichst hochwertiges Laminat sein, was die Gasdurchlässigkeit betrifft. Wie die Untersuchungen jedoch gezeigt haben, können bereits mit einem handelsüblichen, einfachen Aluminium-Laminat mit Polyethylen-Beschichtung sehr gute Lagerstabilitäten erzielt werden.

Ein erster Volumsanteil 4 wird von einer tonometrierten Flüssigkeit beansprucht, welche zumindest eine gelöste Gaskomponente enthält. Weiters ist im flexiblen Behälter 1 eine einen zweiten Volumsanteil 5 beanspruchende Gasphase vorgesehen, welche zumindest die in der Flüssigkeit gelöste Gaskomponente enthält. Der die beiden Volumsanteile 4 und 5 trennende Flüssigkeitsspiegel ist mit 6 gekennzeichnet. Zur Entnahme der Flüssigkeit ist ein Schlauch 7, vorzugsweise aus Polyethylen, im Beutel vorgesehen. Der Anschluss kann von jeder seitlichen Position erfolgen. Bevorzugt wird jedoch die obere Position, im Bereich des verschweißten Randes 3, damit beim Öffnen und Anschließen des Beutels keine Flüssigkeit austritt. Die Ankopplung muss möglichst gasdicht erfolgen, um die Stabilität der Reagenzien im eingesetzten Zustand sicherzustellen

Bei der Befüllung des flexiblen Behälters kann gemäß Fig. 2 eine begrenzende Vorrichtung 8, beispielsweise eine Außenkonturform verwendet werden, welche die Entfaltung des Behälters bei einem vorgegebenen Volumen begrenzt. Nach Abnahme der begrenzenden Vorrichtung 8 zeigt der flexible Beutel die aus Fig. 1 ersichtliche äußere Gestalt, wobei das durch die Begrenzungseinrichtung vorgegebene Volumen 30-90%, vorzugsweise 60-75% des maximalen Füllvolumens beträgt. Die tonometrierte Flüssigkeit sowie die Gasphase enthalten beispielsweise zumindest eine Gaskomponente aus der Gruppe O₂, CO₂ und N₂.

Eine tonometrierte Flüssigkeit, welche beispielsweise in der Blutgasanalytik Verwendung findet, weist beispielsweise folgende Gaskonzentrationen auf:

cO₂ = 12%, cCO₂ = 6%, cN₂ = 82%

Dies bedeutet für die lagerungstechnisch kritische Gaskomponente O₂, dass z.B. in einem Füllvolumen von 200 ml wässriger Lösung bei 25°C ohne Gasphase gemäß Stand der Technik nur 0,62 ml Sauerstoff gelöst vorliegen. Werden die 200 ml Füllvolumen auf 100 ml wässerige Lösung und 100 ml Gasvolumen aufgeteilt, dann erhöht sich in diesem Beispiel das Sauerstoffvolumen auf 12,31 ml, also um ca. um das zwanzigfache, wodurch sich die guten Lagereigenschaften erklären lassen.

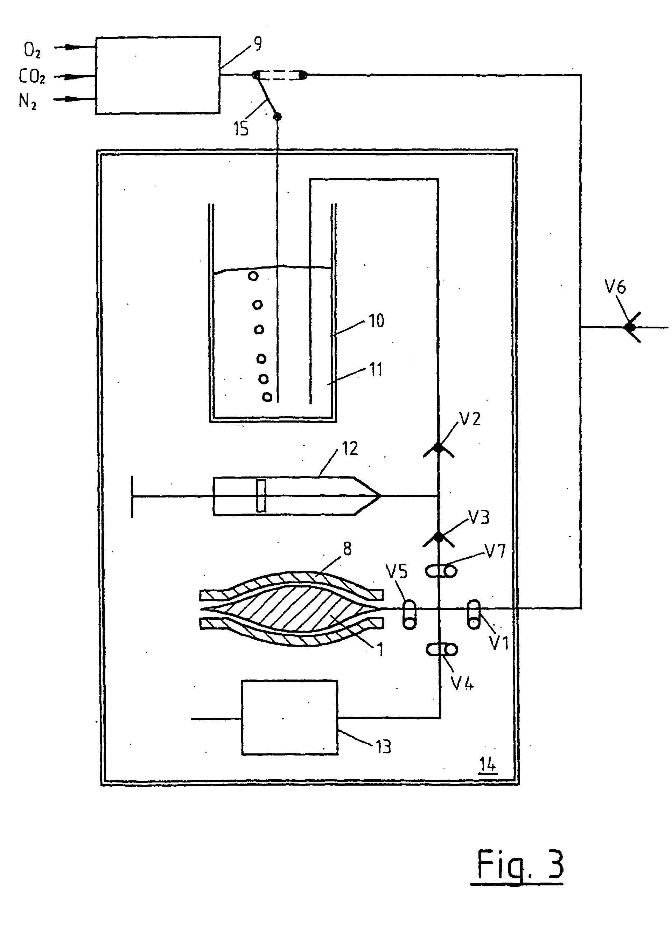

Fig. 3 zeigt das Beispiel einer Abfüllanlage für den flexiblen Behälter 1, welche vorteilhafterweise aus einem Hochpräzisionsgasmischer 9, einem Tonometriergefäß 10 für die abzufüllende Flüssigkeit 11, einer Dosiervorrichtung 12, einer Aufnahme (bzw. begrenzenden Vorrichtung 8) für den flexiblen Behälter 1, einer Vakuumpumpe 13, sowie einer Vorrichtung 14 zur Thermostatisierung der einzelnen Komponenten besteht.

Die Abfüllung erfolgt in drei Schritten:
1. Schritt: Begasung des zu füllenden flexiblen Behälters mittels eines Präzisionsgases, welches im Gasmischer 9 aus den Primärgasen O₂, CO₂ und N₂ erzeugt und vorteilhafterweise auch für die Tonometrie der abzufüllenden Flüssigkeit 11 im Tonometriergefäß 10 verwendet wird.
   Bei diesem Schritt steht das Zweiwegventil 15 in der strichlierten Position und die Ventile V1 und V5 sind offen, das Ventil V4 geschlossen. Mit dem Überdruckventil V6 wird dabei sichergestellt, daß es zu keiner Beschädigung des flexiblen Behälters 1 kommt. Anschließend wird der flexible Behälter über die Vakuumpumpe 13 evakuiert, wobei die Ventile V1, V7 geschlossen und die Ventile V4, V5 geöffnet sind.
2 . Schritt: Befüllung des flexiblen Behälters 1 mit der Dosiervorrichtung 12.
   Als vorteilhaft hat es sich erwiesen, die Einkopplung der Dosiervorrichtung 12 über zwei Rückschlagventile V2 und V3 vorzunehmen, wobei die Dosiervorrichtung über das Ventil V2 eine vorgegebene Flüssigkeitsmenge aus dem Tonometriergefäß 10 ansaugt und über das Rückschlagventil V3 an den flexiblen Behälter 1 abgibt.
3 . Schritt: Einbringung eines definierten Gasvolumens des Präzisionsgases.
   Der flexible Behälter 1 wird nach dem Umschalten des Zweiwegventiles 15 in die strichlierte Position mit Präzisionsgas gefüllt, bis sich der flexible Behälter an die Wand der Aufnahme 8 anschmiegt und das Überdruckventil V6 öffnet.

Nach dem Beenden des Füllvorganges und nach dem Schließen des Ventils V5 kann der flexible Behälter aus der Aufnahme 8 entnommen und der Beutelanschluss verschlossen werden.

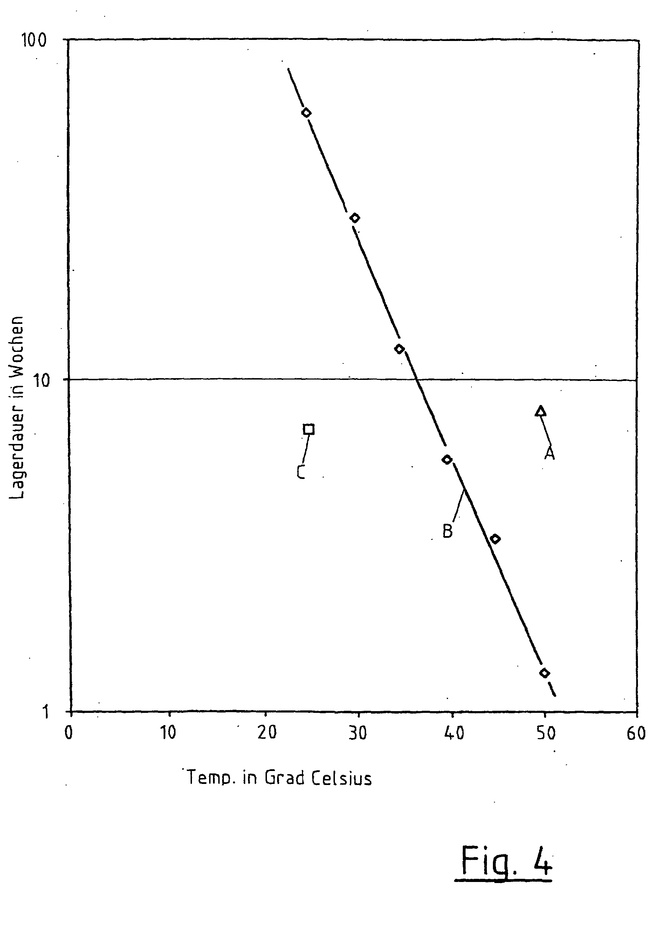

Fig. 4 zeigt ein Diagramm, bei welchem in Abhängigkeit der Temperatur in °C die Lagerdauer in Wochen aufgetragen wurde, wobei eine Abweichung der Ausgangswerte um 2% zugelassen wurde. Eine Elektrolytlösung A mit einem Bikarbonatpuffer konnte bei Anwendung des erfindungsgemäßen Verfahrens 8 Wochen bei 50°C gelagert werden, wobei eine Abweichung von 2% gegenüber der gekühlt gelagerten Elektrolytlösung auftrat. Die Kurve B (Daten aus WO97/16309) und der Messpunkt C (Mallinckrodt Sensor Systems Inc.) zeigen die im Vergleich zur Erfindung beschränkte Lagerfähigkeit von tonometrierten Flüssigkeiten gemäß Stand der Technik.

## Patentansprüche

1. Verfahren zur Verbesserung der Lagerfähigkeit tonometrierter Flüssigkeiten mit zumindest einer gelösten Gaskomponente, wobei die tonometrierte Flüssigkeit in einen vor dem Befiillen kollabierten, beim Befüllen sich entfaltenden flexiblen Behälter (1), vorzugsweise einen Aluminium-Mehrschichtlaminatbeutel (2) gefüllt wird, und wobei in den flexiblen Behälter (1) zusätzlich eine zumindest die gelöste Gaskomponente enthaltende Gasphase eingebracht wird, **dadurch gekennzeichnet, dass** die Gasphase von der tonometrierten Flüssigkeit durch deren Flüssigkeitsspiegel (6) getrennt ist und wobei das Volumen der tonometrierten Flüssigkeit (4) und das Volumen der Gasphase (5) in Summe kleiner ist als das maximale Füllvolumen des flexiblen Behälters.

2. Verfahren nach Anspruch 1, **dadurch gekennzeichnet, dass** der flexible Behälter (1) in eine die Entfaltung des Behälter bei einem vorgegebenen Volumen begrenzende Vorrichtung eingesetzt wird, dass eine abgemessene Menge der tonometrierten Flüssigkeit in den Behälter gefüllt wird und dass anschließend die Gasphase eingebracht wird, wobei sich der flexible Behälter bis zum vorbestimmten Volumen entfaltet.

3. Verfahren nach Anspruch 2, **dadurch gekennzeichnet, dass** das vorgegebene Volumen 30-90%, vorzugsweise 60-75% des maximalen Füllvolumens beträgt.

4. Eine tonometrierte Flüssigkeit mit zumindest einer gelösten Gaskomponente enthaltender Behälter (1) aus einem flexiblen, gasdichten Material, welcher sich beim Befüllen entfaltet und beim Entleeren kollabiert, vorzugsweise ein Aluminium-Mehrschichtlaminatbeutel (2), wobei die tonometrierte Flüssigkeit einen ersten Volumsanteil (4) beansprucht und der flexible Behälter (1) eine einen zweiten Volumsanteil (5) beanspruchende Gasphase aufweist, welche zumindest die in der Flüssigkeit gelöste Gaskomponente enthält, **dadurch gekennzeichnet, dass** die Gasphase von der tonometrierten Flüssigkeit durch deren Flüssigkeitsspiegel (6) getrerint ist, sowie dass der erste (4) und der zweite Volumsanteil (5) in Summe kleiner sind als das maximale Füllvolumen des flexiblen Behälters.

5. Behälter nach Anspruch 4, **dadurch gekennzeichnet, dass** in der tonometrierten Flüssigkeit sowie in der Gasphase zumindest eine Gaskomponente aus der Gruppe O₂, CO₂ und N₂ enthalten ist.

6. Behälter nach Anspruch 4 oder 5, **dadurch gekennzeichnet, dass** das Verhältnis der Volumsanteile (4, 5) der tonometrierten Flüssigkeit und der Gasphase im Bereich von 1:3 bis 3:1, vorzugsweise bei ca. 1:1, liegt.

## Claims

1. Method of improving the shelflife of tonometered fluids with at least one dissolved gas component, wherein the tonometered fluid is packaged in a flexible container (1) that is collapsed when it is empty but unfolding when it is being filled and is preferably constituted by a multilayered aluminium laminate bag (2), and wherein a gas phase containing at least the dissolved gas component is additionally introduced into the flexible container (1), **characterized in that** the gas phase is separated from the tonometered fluid by the liquid level (6) of the latter, the volume of the tonometered fluid (4) and the volume of the gas phase (5) together amounting to less than the maximum filling volume of the flexible container.

2. Method according to claim 1, **characterized in that** the flexible container (1) is inserted into a receptacle restricting the unfolding of the container to a predefined volume, and that a metered amount of the tonometered fluid is filled into the container, and that the gas phase is subsequently introduced, upon which the flexible container unfolds until the predefined volume is reached.

3. Method according to claim 2, **characterized in that** the predefined volume amounts to 30-90%, and preferably 60-75% of the maximum filling volume.

4. Container (1) containing a tonometered fluid with at least one gas component dissolved therein, which is made from flexible, gas-tight material and unfolds when it is being filled and collapses when it is being drained, preferably a multilayered aluminium laminate bag 2, wherein the tonometered fluid occupies a first part (4) of the volume, and the flexible container (1) holds a gas phase occupying a second part (5) of the volume, which gas phase contains at least the gas component dissolved in the fluid, **characterized in that** the gas phase is separated from the tonometered fluid by the liquid level (6) of the latter, and that the first (4) and second (5) volume parts taken together amount to less than the maximum filling volume of the flexible container.

5. Container according to claim 4, **characterized in that** the tonometered fluid and the gas phase contain at least one gas component from the group of O₂, CO₂, N₂.

6. Container according to claim 4 or 5, **characterized in that** the ratio of the volume parts (4, 5) of the tonometered fluid and the gas phase is between 1:3 and 3:1, and preferably about 1:1.

## Revendications

1. Procédé pour améliorer la stabilité au stockage de fluides tonométriques, avec au moins un composant gazeux dissous, dans lequel le fluide tonométrique est versé dans un récipient (1) replié sur lui-même avant le remplissage, flexible et se dépliant lors du remplissage, de préférence un sachet en stratifié multicouches d'aluminium (2), et dans lequel on introduit dans le récipient flexible (1) en plus une phase gazeuse contenant au moins le composant gazeux dissous, **caractérisé en ce que** la phase gazeuse est séparée du liquide tonométrique par son interface liquide (6) et dans lequel le volume du fluide tonométrique (4) et le volume de la phase gazeuse sont de somme inférieure au volume de remplissage maximal du récipient flexible.

2. Procédé selon la revendication 1, **caractérisé en ce que** le récipient flexible (1) est mis en oeuvre dans un dispositif limitant le dépliage du récipient pour un volume prédéterminé, **en ce qu'**une quantité mesurée de fluide tonométrique est remplie dans le récipient et **en ce que**, ensuite, la phase gazeuse est introduite, le récipient flexible se dépliant jusqu'au volume prédéterminé.

3. Procédé selon la revendication 2, **caractérisé en ce que** le volume prédéterminé va de 30 à 90 %, de préférence de 60 à 75 % du volume de remplissage maximal.

4. Fluide tonométrique avec au moins un récipient (1), contenant un composant gazeux dissous, constitué d'un matériau flexible, étanche au gaz, qui se déplie lors du remplissage et se replie sur lui-même lors de la vidange, de préférence un sachet en stratifié multicouches d'aluminium (2), dans lequel le fluide tonométrique revendique un premier pourcentage volumique (4) et le récipient flexible (1) présente une phase gazeuse revendiquant un deuxième pourcentage volumique (5), qui contient au moins le composant gazeux dissous dans le fluide, **caractérisé en ce que** la phase gazeuse est séparée du fluide tonométrique par son interface liquide (6), et **en ce que** le premier (4) et le deuxième pourcentage volumique (5) sont de somme inférieure au volume de remplissage maximal du récipient flexible.

5. Récipient selon la revendication 4, **caractérisé en ce qu'**au moins un composant gazeux issu du groupe O₂, CO₂ et N₂ est présent dans le fluide tonométrique ainsi que dans la phase gazeuse.

6. Récipient selon la revendication 4 ou 5, **caractérisé en ce que** le rapport des pourcentages volumiques (4, 5) du fluide tonométrique et de la phase gazeuse se situe dans l'intervalle allant de 1 :3 à 3 :1, de préférence vers 1 :1.
